# EUROPEAN PATENT SPECIFICATION

(11) **EP 2 165 733 B1**
(45) Date of publication and mention of the grant of the patent: **23.04.2014**
(21) Application number: 08790615.2
(22) Date of filing: 25.06.2008
(51) Int. Cl.: A61N 1/36, A61H 1/02, A61H 23/02, A61N 1/04

(54) **WEAR FOR ELECTRICALLY STIMULATING MUSCLES**
KLEIDUNGSSTÜCK ZUR ELEKTRISCHEN MUSKELSTIMULATION
VÊTEMENT CONÇU POUR STIMULER ÉLECTRIQUEMENT DES MUSCLES

(30) Priority: 27.06.2007 JP 2007169554
(43) Date of publication of application: 24.03.2010
(73) Proprietor: Kurume University, Kurume-shi Fukuoka 8300011 (JP); Kyushu Institute of Technology, Kitakyushu-shi, Fukuoka 804-8550 (JP); Japan Aerospace Exploration Agency, Chofu-shi, Tokyo 182-8522 (JP); Japan Space Forum, Tokyo 100-0004 (JP)
(72) Inventor: SHIBA, Naoto, Kurume-shi Fukuoka 830-0011 (JP); YAMAGUCHI, Hitomi, Kurume-shi Fukuoka 839-0863 (JP); TAGAWA, Yoshihiko, Kitakyushu-shi Fukuoka 804-8550 (JP); NUMADA, Kiyoshi, Oyabe-shi Toyama 932-0193 (JP); YOSHIKAWA, Kazuhiro, Tenri-shi Nara 632-8505 (JP); MURAKAMI, Keiji, Tsukuba-shi Ibaraki 305-8505 (JP); TAKEOKA, Hajime, Tokyo 100-0004 (JP)
(74) Representative: Isarpatent
(86) International application number: PCT/JP2008/061548
(87) International publication number: WO 2009/001858

(56) References cited:
- GB-A- 2 393 396
- JP-A- 07 289 649
- JP-A- 09 503 937
- JP-A- 60 005 170
- JP-A- 2000 316 988
- JP-A- 2002 534 231
- US-A- 4 583 547
- US-A- 4 919 148
- US-A1- 2007 049 814

## Description

### TECHNICAL FIELD

The present invention relates to a garment for electrically stimulating muscles. More specifically, the present invention relates to a garment used for rehabilitation or exercise, and when electrical muscle stimulation (electro-stimulation of skeletal muscles) to train muscles using muscular contraction by electrically stimulating the muscles is conducted, the electrode positions can be easily determined so that the electrodes are set up at a position where the neuromuscular junction of the muscle to be moved can be stimulated.

### BACKGROUND ART

Electrical muscle stimulation (EMS) has drawn attention as an effective method that can be applied to equipment to assist in rehabilitation and exercise equipment (including so-called health equipment). Exercise based on electrical muscle stimulation differs from exercise using heavy objects such as lifting dumbbells, and has been demonstrated to be a safer exercise method that trains muscles using muscular contraction by electrically stimulating the muscles.

This kind of equipment provides electrodes for electrically stimulating muscles. The positions of the electrodes on the body of the wearer are normally adjusted or set up based on the experience of the wearer or of an assistant.
Further, movement using electrical muscle stimulation does not mean that electrical stimulation may stimulate any location or position of the muscle, but rather in order to have a full movement effect it is extremely important to set up the electrodes in positions that can stimulate the neuromuscular junction (part where the motor nerve joins the muscle: motor point) of the muscle.

The apparatus for strengthening muscle in JP 200279536A proposed by the present applicant is one example of the aforementioned exercise equipment. This apparatus for strengthening muscles includes a training harness comprising an electrode unit that electrically stimulates the muscles and causes contraction, a support harness that works together with the training harness, a sensor to detect a change of an angle formed between the training harness and the support harness from a specified angle, and a controller to supply electricity to the electrode unit. The apparatus is adapted to supply electricity to the electrode unit upon a detection event of the sensor, to electrically stimulate a muscle that is a main motor muscle or a muscle that is an antagonist muscle.

Document JP 2000316988 discloses a garment having the features of the preamble of claim 1.

### DISCLOSURE OF THE INVENTION

### PROBLEMS TO BE RESOLVED BY THE INVENTION

In typical exercise equipment that adopts the method of exercise based on electrically stimulating muscles, it is necessary to search for the exact positions when positioning the electrodes to stimulate the neuromuscular junctions of the muscles. There is a problem that complicated and time consuming operations and manipulations are required when the wearer or assistant carries out the operations and manipulations to search for these positions based on experience as described above.

Moreover, the aforementioned apparatus for strengthening muscles of JP2000279536A is capable of electrically stimulating an antagonist muscle while the main muscle is in a contracted state, so that the antagonist muscle can be exercised in accordance with a closed movement chain associated with centrifugal contraction, which is very useful and practical in terms of being able to conduct effective exercise.

Nonetheless, the apparatus for strengthening muscles described above has a structure that provides a training harness containing the electrode unit, and a support harness that works together with it. Because the positions of the electrodes have been already determined on the training harness, the positions of the electrodes in relation to the body are determined by the mounting position of the training harness. For this reason it is difficult to make fine adjustments of the electrode positions.

Thus, an object of the present invention is to offer a garment for electrically stimulating muscles, for example for use as rehabilitation or exercise equipment, that enables simple electrode position determination such that the electrodes are set at positions where the neuromuscular junctions of the muscles targeted for movement can be stimulated, when conducting electrical stimulation of muscles to train muscles utilizing muscle contraction by electrically stimulating the muscle.

### MEANS TO RESOLVE THE PROBLEMS

The present invention is a garment as defined by claim 1.

Preferably, the electrode is an adhesive seal electrode that can contact the connection terminal and can adhere to the surface of the skin through a mesh opening of the aforementioned mesh.

Preferably the present invention provides an electric cord, one end of which is connected to the electrodes and the other end of which can be connected to an electrical muscle stimulation device.

Preferably, the garment main body of the present invention comprises a clothing-shaped upper half body garment, a clothing-shaped lower half body garment, or both.

In order to set the electrodes at accurate positions at the time of wearing, that is on the surface of the skin in the vicinity of the neuromuscular junction of the muscle, more preferably the garment main body is a garment that inevitably determines the positions of the various parts of the garment in relation to the body of a wearer through the wearers' wearing the garment main body, such as, for example, the upper body garment or lower body garment of a wet suit.

With this kind of garment, even if the positions of the openings and electrodes are determined in advance, the inconvenience of discrepancies occurring in the positions of the electrodes in each wearing is less prone to occur.
Moreover, besides so-to-be-called clothing structures such as the upper body garment or lower body garment, structures locally mountable on the foot (leg) or hand (arm) like a supporter, for example, may be adopted as the garment main body in the present invention.

Preferably, the electrode securing member is provided to cover or close off the opening, but this is not a limitation. The electrode securing member enables to conduct electricity to the skin by allowing the electrode to be secured to pass from the front surface through to the back surface of the electrode securing member, or by connecting the electrode from the front side of the electrode securing member to a conductor provided on part of the back side. Preferably netting (including a mesh, a net or the like) provided with this functionality is adopted as the electrode securing member, but this is not a limitation. For example, cloth, paper, or sheets or film of a variety of synthetic resins provided with holes may also be adopted for the electrode securing member.

When an electrode conducts electricity to the skin, preferably the electrode securing member is formed by a non-conductor, but may also be formed by a conductor. If the electrode securing member is formed by a conductor, then the entirety of the electrode securing member contacting the skin will act as an electrode in the same way as the electrode to be secured, and therefore the size and shape must be adapted for conducting electricity to the skin, specifically, for stimulating the neuromuscular junction of the muscle, and thus more accurate position determination is required.

The connecting terminals and electrodes, while not particularly limited with regard to materials, shapes and structures as long as electricity can be conducted to the skin without obstruction, are formed using a specified conductive substance. This kind of conductive substance may include, for example, metals such as metal plate, metal wire (including stranded wire), silver, silver-silver chloride compound, nickel, and molybdenum, conductive paste prepared using carbon black or graphite singly or in a mixture of 2 or more kinds; laminates of metal foil such as silver or of metal fibers such as stainless steel fibers; or cloth woven with threads containing metal fibers. Specifically, cloth woven with thread containing metal fibers is flexible and able to conform to the shape of the body, and therefore is more preferable because this cloth allows contact with the curved surface of the skin without the wearer feeling discomfort.

As long as adhesive seal electrodes have satisfactory adhesion to the surface of the skin, their materials and structure are not particularly limited. For example, an adhesive seal electrode formed by affixing a conductive adhesive tape material, an adhesive seal material, or a conductive adhesive material on a substrate may be adopted. Specifically, an adhesive seal electrode with a structure that includes a substrate sheet, an electro-conductive layer capable of conducting electricity formed using a conductive substance on the surface of the substrate sheet, and a conductive adhesive material layer provided to make contact with this conductive layer is preferable because the conductive adhesive material layer provides satisfactory adhesion to the surface of the skin, and the contact impedance between the surface of the skin may be reduced since the input area conducting electricity to the skin can be increased. Moreover, the adhesive seal electrode has the function to enable the electric current that is conducted to the connection terminal unit to pass from the connection terminal through the conductive adhesive material, to spread over the entire surface of the conductive layer formed on the surface of the substrate sheet, and further to be conducted to the surface of the skin through the conductive adhesive material layer.

Although the aforementioned substrate sheet is not particularly limited, preferably a flexible resin film or sheet with comparatively high tear strength is adopted in order to allow close adhesion of the conductive adhesive material layer to the surface of the skin. For example, polyethylene terephthalate (PET), polyvinyl chloride, polyethylene (PE), polypropylene (PP) and the like may be adopted as this kind of resin film or sheet, but polyethylene terephthalate, which has high tear strength and is easily printed on, is more preferable. Further, the thickness of the substrate sheet is not particularly limited, but may be suitably set, for example, at 10 to 500 µm.

The aforementioned conductive layer formed on the surface of the substrate sheet is not particularly limited as long it can be implemented without interfering with the flow of electricity, and may be formed using a specified conductive substance. Examples of this kind of conductive substance include metals such as silver, silver-silver chloride compounds, nickel, and molybdenum, or conductive pastes prepared using carbon black or graphite singly or in a mixture of two or more kinds. Moreover, the electrode unit may be formed by making laminates of metal foil such as silver or of metal fibers such as stainless steel fibers.

The aforementioned conductive adhesive material layer is not particularly limited as long as the layer is conductive, does not irritate the skin excessively, and has sufficient adhesive force. Optimally, a conductive adhesive gel is used in which water and electrolytic salt and the like are added to a matrix of a polyacrylate derivative such as sodium polyacrylate, ester polyacrylate or polyacrylamide, or of a poly-N-vinyl acetamide derivative such as polyvinylpyrrolidone or poly-N-vinyl acetamide, or of polyurethane and the like.

### (Action)

The action of the garment for electrically stimulating muscles related to the present invention will be explained. Herein, configurational elements used in the explanation are referred to by symbols in correspondence with symbols assigned to the various parts in the embodiments to be described later. However, in the same way as the symbols included in the claims, these symbols are ultimately for the purpose of simplifying the understanding of the content, and the meanings of the configurational elements are not limited to those of the parts of the embodiments.

By a wearer's putting on the garment (A1, A2) for electrically stimulating muscles, electrodes (8) provided in an opening (2, 2a) are positioned on the surface of the skin at the neuromuscular junctions or in the vicinity of the neuromuscular junctions of the muscles of the wearer targeted for exercise. Then, exercise by electrical muscle stimulation (EMS) can be carried out by conducting electricity to the skin through the electrode (8) using electrical muscle stimulation equipment.

In this way, because the electrodes (8) are installed beforehand in the aforementioned specified positions of the garment (A1, A2), when electrical muscle stimulation is carried out in order to train the muscles by taking advantage of the fact that electrical stimulation of a muscle causes the muscle to contract, it is not necessary to adjust the position of the electrodes (8) at each time of use, and position determination of the electrodes (8) can be simply conducted just by putting on the garment (A1, A2).

Because a garment that provides an electrode securing member (3), which is formed by non-conductive material and can secure the electrodes (8) provided in the openings (2, 2a), enables adjustment of the positions for securing the electrodes (8) during manufacture or after manufacture of the garment, this type of garment is particularly useful when, for example, the garment (A1, A2) is divided into multiple types corresponding to the body types of wearers.

Further, when the electrode securing member (3) is netting providing multiple mesh openings, the electrodes (8) can make contact with the surface of the skin through the mesh openings even when the electrode is secured to the top surface side of the netting. Moreover, the netting has satisfactory air circulation, and has the advantage that the surface of the skin where the electrode makes contact is less prone to become sweaty.

A garment in which the electrode securing member is netting (3) and in which the electrodes are adhesive seal electrodes (8) that can make contact with connection terminal (4) and adhere to the surface of the skin through the aforementioned mesh openings of the netting (3) is preferable because the input surface area conducting electricity to the skin can be increased, and therefore the contact impedance with the surface of the skin is reduced.

A garment that provides an electric cord (5) of which one end is connected to the terminals (8) and the other end can connect to an electrical muscle stimulation device (C1) enables exercise by electrical muscle stimulation to be easily conducted by connecting the electrical muscle stimulation device (C1) to the electric cord (5).

By the wearer wearing a garment main body (1) comprising a clothing-shaped upper body garment, lower body garment, or both, the positions of each part of the garment (A1, A2) are necessarily determined in relation to the body of the wearer so that even if the positions of the openings (2, 2a) and electrodes (8) are determined in advance, the inconvenience of the positions of the electrodes becoming misaligned in each wearing is less prone to occur.

### EFFECTS OF THE INVENTION

The present invention is usable, for example, as a garment for rehabilitation and exercise and enables to provide a garment for electrically stimulating muscles in which, when conducting electrical muscle stimulation to train muscles by taking advantage of the fact that electrical stimulation of a muscle causes that muscle to contract, electrode positions can be easily determined such that the electrodes are set up in positions to stimulate the neuromuscular junctions of the muscles.

### BRIEF DESCRIPTION OF THE DRAWINGS

FIG. 1 is a front view diagram indicating a first embodiment of a garment for electrically stimulating muscles related to present invention;
FIG. 2 is a back view diagram of the garment for electrically stimulating muscles in FIG. 1;
FIG. 3 is an expanded explanatory diagram indicating the assembly structure of an opening with netting and connecting terminals;
FIG. 4 is an end surface explanatory diagram of a portion marked I-I in FIG. 3;
FIG. 5 is an explanatory diagram indicating a state when an adhesive seal electrode is affixed to the connecting terminal and a netting portion;
FIG. 6 is a cross-sectional explanatory diagram indicating the adhesive seal electrode;
FIG. 7 is an explanatory diagram indicating a state in use;
FIG. 8 is a front view diagram indicating a second embodiment of a garment for electrically stimulating muscles related to present invention; and
FIG. 9 is a back view diagram of the garment for electrically stimulating muscles in FIG. 8.

### Legend

- A1: Garment for electrically stimulating muscles
- 1: Garment main body, 10 Torso part, 11, 12 Sleeve
- 2, 2a: Opening, 20 Piping, 21 Stitching
- 3: Mesh
- 4: Connecting terminal, 40 Sewed part
- 5: Electric cord, 50 terminal
- 6: Pass-through loop
- 8: Adhesive seal electrode, 80 Substrate sheet,
- 81: Conductive layer, 82 Conductive adhesive material
- 9: Fastener
- B: Belt
- C1: Electrical muscle stimulation device, C2 Controller
- M: Wearer
- A2: Garment for electrically stimulating muscles
- 7: Garment main body, 70 Hip part, 71 Leg part

### BEST MODE FOR CARRYING OUT THE INVENTION

The present invention will be explained in detail based on the embodiments indicated in the drawings.

### EMBODIMENT 1

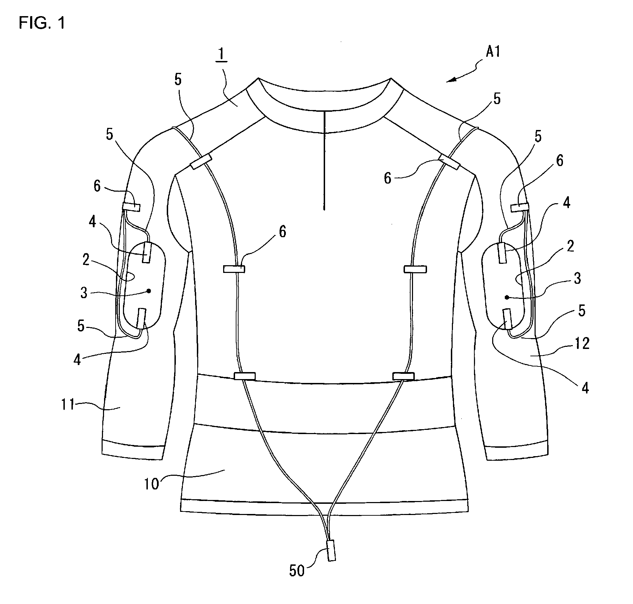

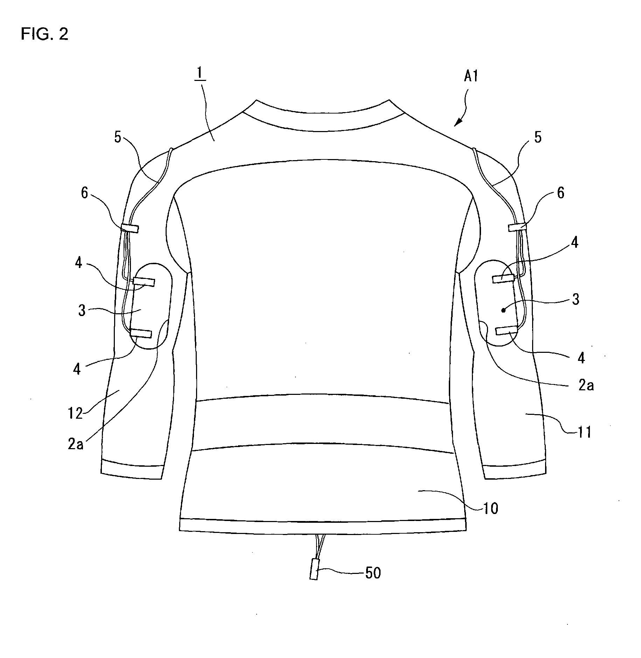

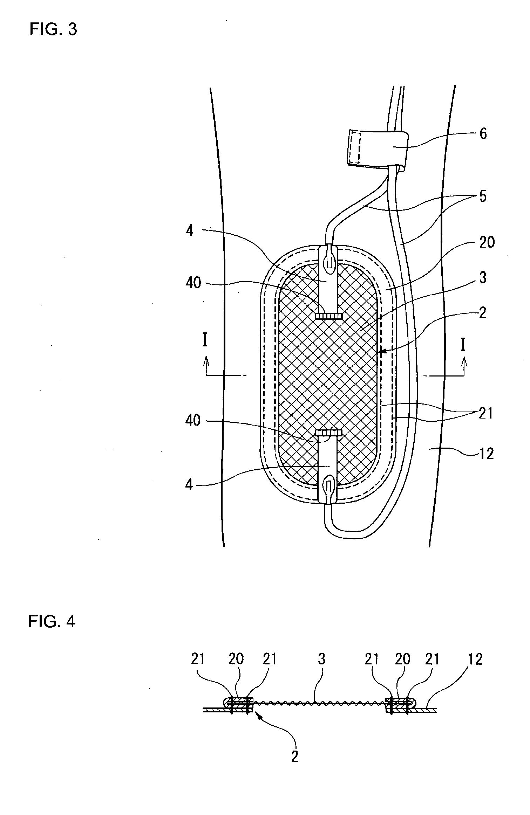

FIG. 1 is a front view diagram indicating a first embodiment of a garment for electrically stimulating muscles related to present invention; FIG. 2 is a back view diagram of the garment for electrically stimulating muscles in FIG. 1; FIG. 3 is an expanded explanatory diagram indicating an assembly structure of an opening with netting and connecting terminals; and FIG. 4 is an end surface explanatory diagram of a portion marked I-I in FIG. 3.

Further, indication of an adhesive seal electrode 8 is omitted from FIG. 1, FIG. 2, FIG. 3, and FIG. 4, respectively. Moreover, indication of electric cords is omitted from FIG. 4.

The garment A1 for electrically stimulating muscles has a garment main body 1. The garment main body 1 is a clothing-shaped garment for the upper body. The garment main body 1 is a non-conductor (insulator), and is formed from cloth having slight elasticity.
Openings 2, 2a have nearly oval edge shapes, and are provided respectively on the front and back sides of the upper arms part of sleeves 11, 12 that are provided on both sides of a torso part 10 of the garment main body 1. Each opening part 2 on the front side is open so as to correspond to the brachial biceps of the upper arm of the wearer when worn, and each opening part 2a on the back side is open so as to correspond to the brachial triceps. Further, the shapes of the openings 2, 2a are not particularly limited to ovals, and a variety of suitable shapes such as circles or squares may be suitably adopted.

A synthetic resin mesh 3 is assembled on the front side edge of the openings 2, 2a (total of four locations) using a cloth piping 20 (refer to FIG. 3). The piping 20 and the fabric of sleeves 11 and 12 are unified by applying stitching 21 following along both sides in the width direction of the piping 20 so that the mesh 3 closes off the openings 2, 2a (air can circulate through visually transparent mesh). Further, the aforementioned material of the piping 20 and the mesh 3 is insulating. The size of the mesh openings of the mesh 3 is not particularly limited as long as the adhesive seal electrodes 8 to be described later can make contact with the surface of the skin of the wearer through the mesh openings.

Connecting terminals 4 are respectively assembled on the front side of the meshes 3 closing off the openings 2, 2a. The connecting terminals 4 are conductive cloth (woven with thread containing metal fiber) formed into rectangles, an insulating sheet (omitted from the diagram) being provided on the entire surface on the side contacting the mesh 3. The connecting terminals 4 are installed symmetrically in two locations on both ends of the longer dimensioned side of the mesh 3 provided on the opening 2 of the front side (refer to FIG. 1 and FIG. 3). Moreover, the connecting terminals 4 are installed in parallel at a required interval at two edge locations on one end of the shorter dimensioned side of the mesh 3 provided on the opening 2a of the back side (refer to FIG. 2).

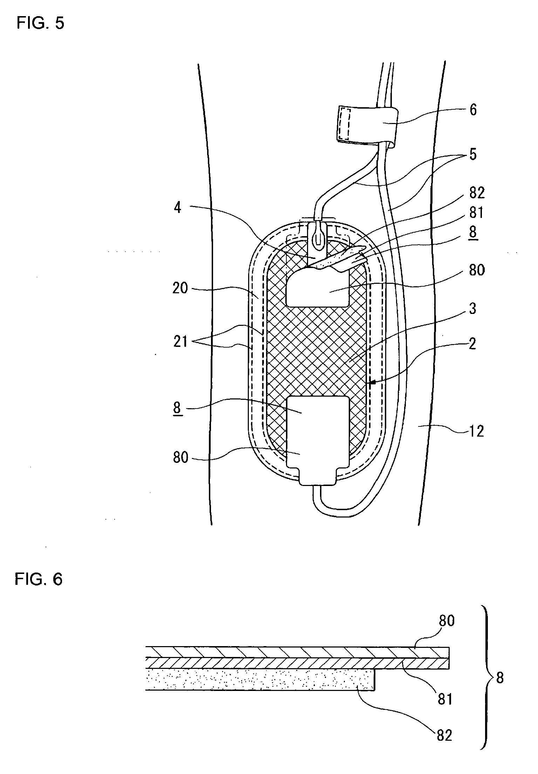

The connecting terminals 4 are secured by sewing one lengthwise end onto the piping 20, the other end being secured to the thread part of the mesh 3 by a sewed part 40. Moreover, one end of the electric cord 5 is connected to the piping side ends of the connecting terminals 4, and the connecting terminals 4 and the garment main body 1 have an insulating relationship (relationship in which electricity is not conducted to the garment main body 1). At the time of use, the adhesive seal electrodes 8 are affixed so as to contact and cover the connecting terminals 4.
As indicated in FIG. 6 to be described later, the adhesive seal electrodes 8 are of a layered form in which a conductive layer 81 and a conductive adhesive material 82 are provided over the entire surface of one side of a substrate sheet 80 formed by a resin film. Electricity passes through the connecting terminals 4 to the conductive adhesive material 82 of the adhesive seal electrode 8, and the conductive adhesive material 82 can adhere tightly and pass electricity to the surface of the skin through the mesh openings of the mesh 3.

The electric cords 5, which extend from the connecting terminals 4 provided in two locations each of the openings 2, 2a, for a total of eight locations, are led from the back sides of sleeves 11 and 12, respectively, around both shoulders to the front side of the torso part 10. The electric cords 5 are bundled at the front side of the torso part 10, and a terminal 50 for connecting to the electrical muscle stimulation device is provided at the end of the cords. Further, the cords 5 pass through pass-through loops 6 provided on the surface of the garment main body 1 at the required locations.

### (Action)

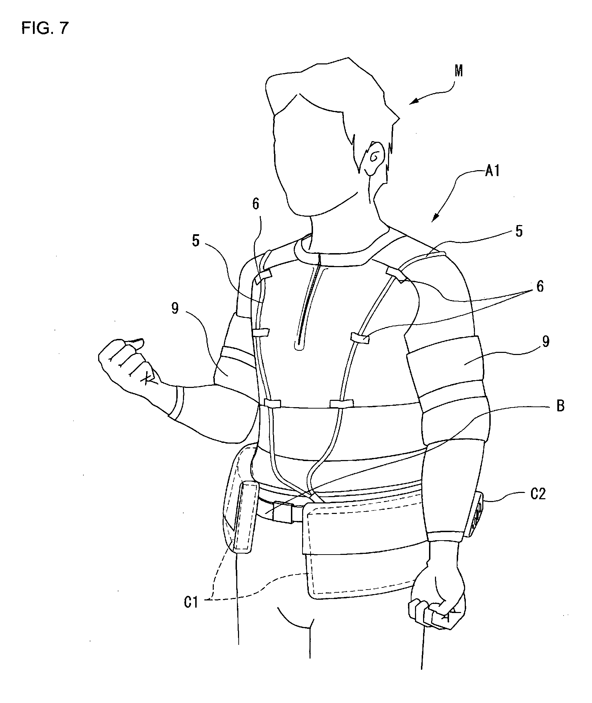

FIG. 5 is an explanatory diagram indicating the state when an adhesive seal electrode is affixed to the connecting terminal and the netting part; FIG. 6 is a cross-sectional explanatory diagram indicating the adhesive seal electrode; and FIG. 7 is an explanatory diagram indicating a state in use.

The garment A1 for electrically stimulating muscles is particularly useful for the wearer to exercise the brachial triceps and brachial biceps of both arms. Moreover, by combining the garment with an electrical muscle stimulation device C1 to control electrical stimulation, exercise combining an agonist and the antagonist muscle in a closed movement chain with centrifugal contraction is possible.

By the wearer M wearing the garment A1 for electrically stimulating muscles, the terminals 4 set up at the openings 2, 2a are positioned in the proximity of the surface of the skin, with the mesh 3 in between, in the vicinity of the neuromuscular junctions of the muscles (brachial triceps and brachial biceps) of the wearer M that are to be exercised. Further, the garment A1 for electrically stimulating muscles is clothing-shaped, and the positions of the various parts of the garment main body 1 are necessarily determined in relation to the body of the wearer M, and therefore even if the positions of the openings 2, 2a and the connecting terminals 4 are determined in advance, the inconvenience of discrepancies occurring in the positions of the adhesive seal electrodes 8 affixed to cover the connecting terminals 4 is less prone to occur between each wearing.

Next, as indicated in FIG. 5, the adhesive seal electrodes 8 are affixed to cover the connecting terminals 4. At this time, by firmly pressing the adhesive seal electrodes 8 onto the mesh 3 side, the conductive adhesive material 82, which makes up the adhesive surface of the adhesive seal electrodes 8, adheres to the surface of the skin through the mesh openings of the mesh 3. Electricity can thereby be conducted over a broad surface area in relation to the surface of the skin via the adhesive seal electrodes 8 that are contacting the connecting terminals 4 through the conductive adhesive material 82, and the adhesive seal electrodes 8 can effectively apply electrical stimuli to the neuromuscular junctions (motor points) of the muscles at these positions.

Further, when exercising, a fastener 9 is wound on the upper arm as indicated in FIG. 7 so that contact between the adhesive seal electrodes 8 and the surface of the skin can be more securely supported. Further, the mesh 3 has satisfactory air ventilation, and there is the advantage that even when the garment is used for a comparatively long time, sweating is not prone to occur on the surface of the skin where the adhesive seal electrode 8 makes contact or on the surface of the skin in that vicinity.

Then, using an electrical muscle stimulation device C1 assembled on the waist of the wearer M with a belt B, exercise can be conducted using electrical muscle stimulation (EMS) by conducting electricity to the surface of the skin through the adhesive seal electrodes 8.
Now, a simple explanation will be given of a method of exercise based on electrical stimulation using the garment A1 for electrically stimulating muscles and the electrical muscle stimulation device C1.

### (1) For movement that causes flexion of the elbow joint

In normal movement that causes flexion from the state of having the elbow joint extended, the brachial biceps becomes the agonist muscle and contracts, while the brachial triceps becomes the antagonist muscle and relaxes. In brief, when movement is going on in this way, electricity is conducted to the surface of the skin from the adhesive seal electrodes 8 set up at the back side openings 2a of the sleeves 11, 12 of the garment A1 for electrically stimulating muscles, without conduction of electricity from other adhesive seal electrodes 8.

Consequently, the brachial triceps does not enter the relaxed state, but contracts to a suitable degree. Specifically, in this movement, the resistance caused by contraction of the brachial triceps, which naturally forms the antagonist muscle, provides a suitable degree of load to train the brachial biceps being the agonist. Because concurrently a load of centrifugal contraction is applied in relation to the brachial triceps, the brachial triceps can be simultaneously trained.

### (2) For movement that causes extension of the elbow joint

In normal movement that causes extension from the state of having the elbow joint flexed, the brachial triceps becomes the agonist and contracts, while the brachial biceps becomes the antagonist muscle and relaxes. In brief, when movement is going on in this way, electricity is conducted to the surface of the skin from the adhesive seal electrodes 8 set up at the front side openings 2 of the sleeves 11, 12 of the garment A1 for electrically stimulating muscles, without conduction of electricity from other adhesive seal electrodes 8.

Consequently, the brachial biceps does not enter the relaxed state, but contracts to a suitable degree. Specifically, in this movement, the resistance caused by contraction of the brachial biceps, which naturally forms the antagonist muscle, provides a suitable degree of load to train the brachial triceps being the agonist. Because concurrently a load of centrifugal contraction is applied in relation to the brachial biceps, the brachial biceps can be simultaneously trained.

Further, a controller C2 for setting up and adjusting control based on the electric muscle stimulation device C1 is installed on the left wrist of the wearer M. Continuous alternating repetition of the aforementioned movements is conducted, switching of the control at the time of each movement being conducted using various sensors. Details of this kind of controller and the control method will be omitted here. The same will be done in the explanation of the action of the garment A2 for electrically stimulating muscles to be described later.

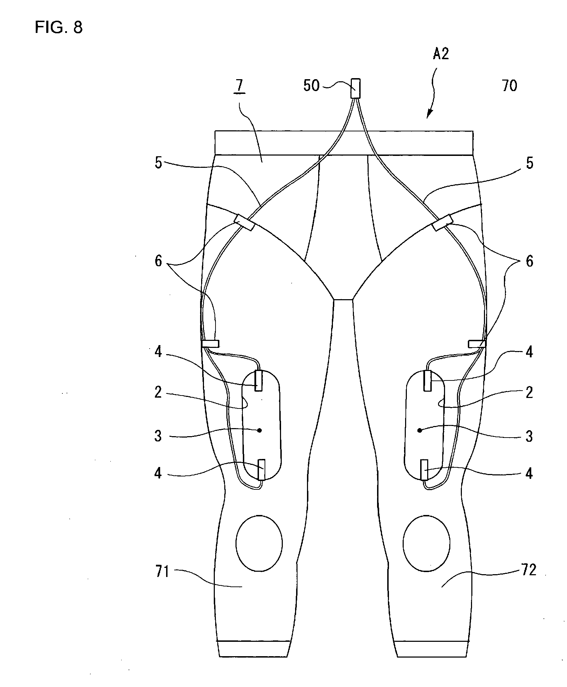

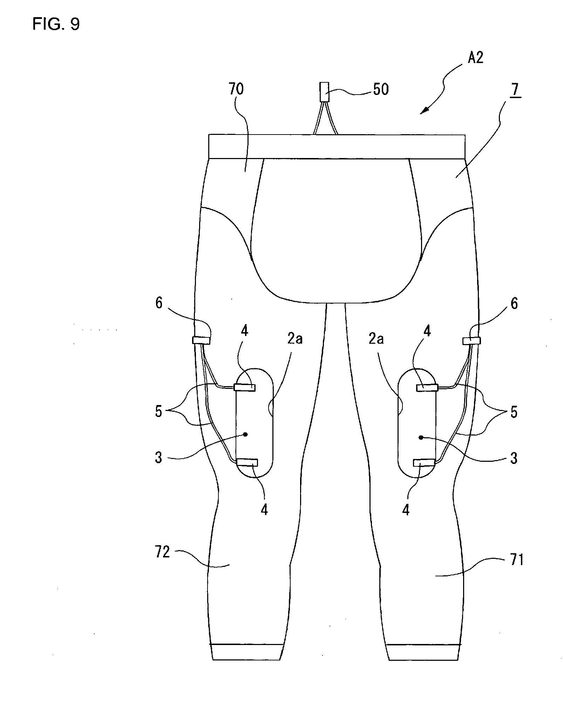

FIG. 8 is a front view diagram indicating a second embodiment of a garment for electrically stimulating muscles related to present invention; and FIG. 9 is a back view diagram of the garment for electrically stimulating muscles in FIG. 8.

Further, in the present embodiment, the same symbols are attached to locations in the diagrams equivalent to those of the aforementioned garment A1 for electrically stimulating muscles, and redundant explanations of the basic structures will be omitted. Moreover, depiction of the adhesive seal electrodes 8 has been omitted in FIG. 8 and FIG. 9.

The garment A2 for electrically stimulating muscles has a garment main body 7. The garment main body 7 is clothing-shaped lower body garment (pants). The garment main body 7 is non-conductive (insulating), and is formed of cloth having slight elasticity.
Openings 2, 2a have nearly oval edge shapes, and are provided respectively on the front and back sides of the thigh part of leg parts 71, 72 that are provided on both sides of a hip part 70 of the garment main body 7. The opening parts 2 on the front side open so as to correspond to the femoral quadriceps of the thigh of the wearer when worn, and opening parts 2a on the back side open so as to correspond to the femoral biceps. Further, the shapes of the openings 2, 2a are not particularly limited to ovals, and a variety of suitable shapes such as circles or squares may be suitably adopted.

The piping 20, mesh 3 and connecting terminals 4, which all have the same structures as in the garment A1 for electrically stimulating muscles described above, are set up on the side edge surface of the openings 2, 2a (a total of four locations). Moreover, one end of the electric cord 5 is connected to the connecting terminals 4 in the same way, and the terminal 50 is set up on the tip of the bundled electric cords 5 in the same way.

### (Action)

A simple explanation will be given of the method of movement based on electrical stimulation using the garment A2 for electrically stimulating muscles and the electrical stimulation device C1.

### (1) For movement that causes flexion of the knee joint

In normal movement that causes flexion from the state of having the knee joint extended, the femoral biceps becomes the agonist muscle and contracts, while the femoral quadriceps becomes the antagonist muscle and relaxes. In brief, when movement is going on in this way, electricity is conducted to the surface of the skin from the adhesive seal electrodes 8 set up at the front side openings 2 of the leg parts 71, 72 of the garment A2 for electrically stimulating muscles, without conduction of electricity from other adhesive seal electrodes 8.

Consequently, the femoral quadriceps does not enter the relaxed state, but contracts to a suitable degree. Specifically, in this movement, the resistance caused by contraction of the femoral quadriceps, which naturally forms the antagonist muscle, provides a suitable degree of load to train the femoral biceps being the agonist. Because concurrently a load of centrifugal contraction is applied in relation to the femoral quadriceps, the femoral quadriceps can be simultaneously trained.

### (2) For movement that causes extension of the knee joint

In normal movement that causes extension from the state of having the knee joint flexed, the femoral quadriceps becomes the agonist and contracts, while the femoral biceps becomes the antagonist muscle and relaxes. In brief, when movement is going on in this way, electricity is conducted to the surface of the skin from the adhesive seal electrodes 8 set up at the back side openings 2a of the leg parts 71, 72 of the garment A2 for electrically stimulating muscles, without conduction of electricity from other adhesive seal electrodes 8.

Consequently, the femoral biceps does not enter the relaxed state, but contracts to a suitable degree. Specifically, in this movement, the resistance caused by contraction of the femoral biceps, which naturally forms the antagonist muscle, provides a suitable degree of load to train the femoral quadriceps being the agonist. Because concurrently a load of centrifugal contraction is applied in relation to the femoral biceps, the femoral quadriceps can be simultaneously trained.

Further, the terminology and expressions used in this Description, which were adopted strictly for explanatory purposes, are not limited in any way, and there was no intention to exclude terminology or expressions equivalent in value to all or part of the characteristics described in this Description. In addition, obviously a variety of modifications of the embodiments is possible within the scope of the present invention, as defined by the claims.

### INDUSTRIAL APPLICABILITY

According to the present invention, a garment for electrically stimulating muscles, usable, for example, as rehabilitation or exercise equipment, can be provided in which determination of electrode positioning can be easily implemented when conducting electrical muscle stimulation to train muscles utilizing the contraction of muscles by electrical stimulation of the muscles, such that the electrodes can be set up at positions that can stimulate the neuromuscular junctions of the muscle to be exercised.

## Claims

1. Garment for electrically stimulating muscles, comprising:
a garment main body (1, 7);
a predetermined number of openings (2, 2a), which are set up in the garment main body (1, 7), so as to be positioned at a neuromuscular junction or
in the vicinity including the neuromuscular junction of a muscle to be exercised when a wearer is wearing said garment main body (1,7);
electrodes (8), which are disposed in said openings (2, 2a) so as to be positioned in the vicinity of the surface of the skin at the neuromuscular junction or in the vicinity of the neuromuscular junction of the muscle when the wearer is wearing said garment main body (1,7); and a mesh (3) which is formed of a non-conductive body, **characterised in that** said mesh (3) is configured to secure the electrodes (8) at an inner position of the openings (2,2a) and has a size of mesh openings such that the electrodes (8) can make contact with the surface of the skin of the wearer through the mesh openings.

2. The garment for electrically stimulating muscles according to Claim 1,
wherein the electrodes are adhesive seal electrodes (8) that are configured to contact connecting terminals (4) and to adhere to the surface of the skin through a mesh opening of said mesh (3).

3. The garment for electrically stimulating muscles according to Claim 1,
further comprising an electric cord (5), of which one end is connected to the electrodes (8) and the other end can be connected to an electrical muscle stimulation device (C1).

4. The garment for electrically stimulating muscles according to Claim 1,
wherein the garment main body (1, 7) comprises a clothing-shaped upper body garment, a clothing-shaped lower body garment, or both.

## Patentansprüche

1. Kleidungsstück zur elektrischen Stimulierung von Muskeln, das Folgendes umfasst:
- einen Kleidungsstück-Hauptkörper (1, 7);
- eine zuvor festgelegte Anzahl von Öffnungen (2, 2a), die in dem Kleidungsstück-Hauptkörper (1, 7) dergestalt ausgebildet sind, dass sie sich an einer motorischen Endplatte eines zu trainierenden Muskels oder in einer Umgebung, welche die motorische Endplatte eines zu trainierenden Muskels enthält, befindet, wenn ein Träger den Kleidungsstück-Hauptkörper (1, 7) trägt;
- Elektroden (8), die so in den Öffnungen (2, 2a) angeordnet sind, dass sie sich in der Nähe der Hautoberfläche an der motorischen Endplatte oder in der Nähe der motorischen Endplatte des Muskels befinden, wenn der Träger den Kleidungsstück-Hauptkörper (1, 7) trägt; und
- ein Maschennetz (3), das aus einem nicht-leitfähigen
Körper besteht,
**dadurch gekennzeichnet, dass** das Maschennetz (3) dafür ausgelegt ist, die Elektroden (8) an einer inneren Position der Öffnungen (2, 2a) zu befestigen, und eine solche Größe der Maschenöffnungen aufweist, dass die Elektroden (8) die Hautoberfläche des Trägers durch die Maschenöffnungen hindurch berühren können.

2. Kleidungsstück zur elektrischen Stimulierung von Muskeln nach Anspruch 1, wobei die Elektroden
Klebesiegelelektroden (8) sind, die dafür ausgelelgt sind, Anschlusskontakte (4) zu berühren und durch eine Maschenöffnung des Maschennetzes (3) hindurch an der Hautoberfläche anzuhaften.

3. Kleidungsstück zur elektrischen Stimulierung von Muskeln nach Anspruch 1, das des Weiteren ein Stromkabel (5) umfasst, das an einem Ende mit den Elektroden (8) verbunden ist und am anderen Ende mit einem elektrischen Muskelstimulationsgerät (C1) verbunden werden kann.

4. Kleidungsstück zur elektrischen Stimulierung von Muskeln nach Anspruch 1, wobei der Kleidungsstück-Hauptkörper (1, 7) ein bekleidungsförmiges Oberkörperkleidungsstück, ein bekleidungsförmiges Unterkörperkleidungsstück oder beides umfasst.

## Revendications

1. Vêtement conçu pour stimuler électriquement des muscles, comprenant :
- un corps principal de vêtement (1, 7) ;
- un nombre prédéterminé d'ouvertures (2, 2a), qui sont mises en place dans le corps principal de vêtement (1, 7) de manière à être positionnées au niveau d'une jonction neuromusculaire ou à proximité, y compris la jonction neuromusculaire d'un muscle devant être exercé lorsqu'un porteur porte ledit corps principal de vêtement (1, 7) ;
- des électrodes (8), qui sont disposées dans lesdites ouvertures (2, 2a) de manière à être positionnées à proximité de la surface de la peau au niveau de la jonction neuromusculaire ou à proximité de la jonction neuromusculaire du muscle lorsque le porteur porte ledit corps principal de vêtement (1, 7) ; et
- un filet (3) qui est formé d'un corps non conducteur, **caractérisé par le fait que** ledit filet est configuré pour fixer les électrodes (8) au niveau d'une position intérieure des ouvertures (2, 2a) et a une taille d'ouvertures de filet de telle sorte que les électrodes (8) peuvent entrer en contact avec la surface de la peau du porteur à travers les ouvertures de filet.

2. Vêtement conçu pour stimuler électriquement des muscles selon la revendication 1, dans lequel les électrodes sont des électrodes d'étanchéité adhésives (8) qui sont configurées pour entrer en contact avec des bornes de
raccordement (4) et adhérer à la surface de la peau à travers une ouverture de filet dudit filet (3).

3. Vêtement conçu pour stimuler électriquement des muscles selon la revendication 1, comprenant en outre un cordon électrique (5), dont une extrémité est liée aux électrodes (8) et l'autre extrémité peut être liée à un dispositif électrique de stimulation de muscles (C1).

4. Vêtement conçu pour stimuler électriquement des muscles selon la revendication 1, dans lequel le corps principal de vêtement (1, 7) comprend un vêtement pour le haut du corps en forme d'habit, un vêtement pour le bas du corps en forme d'habit, ou les deux.
